# DEMANDE DE BREVET EUROPEEN

(11) **EP 0 788 836 A1**
(43) Date de publication de la demande: **13.08.1997**
(21) Numéro de dépôt: 97400255.2
(22) Date de dépôt: 05.02.1997
(51) Int. Cl.: B01J 29/06

(54) **Catalyseur comportant une zéolithe de type structural mazzite et son utilisation en dismutation et/ou transalkylation d'hydrocarbures alkylaromatiques**

(30) Priorité: 09.02.1996 FR 9601606
(71) Demandeur: INSTITUT FRANCAIS DU PETROLE, 92502 Rueil-Malmaison (FR)
(72) Inventeur: Benazzi, Eric, 78360 Montesson (FR); Alario, Fabio, 92200 Neuilly sur Seine (FR)

(57) **Abrégé**

L'invention concerne une zéolithe de type structural mazzite, comprenant du silicium, et au moins un élément T choisi dans le groupe formé par le gallium et l'aluminium, de préférence l'aluminium, dont le nombre d'atomes T extra-réseau est inférieur à 15 % du nombre global d'atomes T présents dans la zéolithe, dont le rapport Si/T atomique global est compris entre 5 et 100, ainsi que sa préparation par extraction d'élément T de la charpente zéolithique, de préférence par désalumination de la charpente, par au moins un traitement hydrothermique suivi d'au moins une attaque acide. L'invention concerne un catalyseur comprenant ladite zéolithe au moins en partie sous forme acide, éventuellement au moins un élément choisi dans l'ensemble formé par les groupes IB et VIII de la classification périodique des éléments et au moins une matrice, et l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et de triméthylbenzènes pour produire des xylènes.

## Description

L'invention concerne une zéolithe de type structural mazzite, comprenant du silicium, et au moins un élément T choisi dans le groupe formé par le gallium et l'aluminium, de préférence l'aluminium, dont le nombre d'atomes T extra-réseau est inférieur à environ 15 % du nombre global d'atomes T présents dans la zéolithe, dont le rapport Si/T atomique global est compris entre 5 et 100, ainsi que sa préparation par extraction d'élément T de la charpente (ou réseau) zéolithique, de préférence par désalumination de la charpente, par au moins un traitement hydrothermique suivi d'au moins une attaque acide. L'invention concerne aussi un catalyseur comprenant ladite zéolithe au moins en partie sous forme acide, éventuellement au moins un élément choisi dans l'ensemble formé par les groupes IB et VIII de la classification périodique des éléments et au moins une matrice, et l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et de triméthylbenzènes pour produire des xylènes.

De nombreux catalyseurs de dismutation et de transalkylation à base de mordénite ont déjà été décrits dans l'art antérieur. La mordénite utilisée possède un réseau microporeux monodimensionnel dont le diamètre des pores est de 7 x 6,5 Å (1 Å = 1 Angström = 1.10⁻¹⁰ m) ("Atlas of Zeolites Structure Types", W.M. Meier et D.H. Olson, 3ème Edition, 1992). Il en est ainsi dans le brevet US-A-3.506.731 où une mordénite sous forme hydrogène est utilisée ainsi que dans la demande de brevet français FR-A-2.367.533. Il en est encore ainsi dans le brevet US-A-3.281.483 qui fait mention de mordénites échangées essentiellement avec des ions argent ou nickel, ou bien dans le brevet US-A-3.780.121 qui fait état d'une mordénite échangée avec des métaux du groupe lB de la classification périodique des éléments et qui est caractérisée par un rapport atomique Si/Al compris entre 6 et 40, ou bien encore dans le brevet US-A-3.629.351 qui concerne également une mordénite contenant des ions des métaux du groupe IB, VA, VIA, IIA et VIII de la classification périodique des éléments.

Plus récemment le brevet US-A-5.210.356 revendique l'utilisation d'un catalyseur de dismutation du toluène comportant une zéolithe oméga modifiée par désalumination et chargée en nickel.

D'autre part, le brevet US-A-5.371.311 nous enseigne qu'un catalyseur comportant une zéolithe oméga, synthétisée en utilisant des cations alcalins et un agent organique comme structurant organique, puis modifiée par calcination sous air, échanges ioniques, calcination en présence de vapeur d'eau et finalement par un traitement par une solution aqueuse d'ions ammonium à bas pH, conduit à des propriétés physico-chimiques supérieures et à des performances catalytiques améliorées dans les réactions de conversion des hydrocarbures et en particulier de dismutation et/ou de transalkylation des alkylaromatiques.

De façon surprenante, une zéolithe de type structural mazzite, au moins en partie, de préférence pratiquement totalement, sous forme acide, et comportant un nombre d'atomes T extra-réseau inférieur à 15% du nombre global d'atomes T présents dans ladite zéolithe conduit, lors de son incorporation dans un catalyseur, à des performances catalytiques, et en particulier des sélectivités, améliorées dans les réactions de dismutation d'hydrocarbures alkylaromatiques tel que le toluène, et/ou de transalkylation d'hydrocarbures alkylaromatiques tels que le toluène et les triméthylbenzènes, par rapport aux catalyseurs de l'art antérieur.

L'invention concerne une zéolithe de type structural mazzite, de préférence la zéolithe oméga, comprenant du silicium et au moins un élément T choisi dans le groupe formé par le gallium et l'aluminium, de préférence l'aluminium, dont le rapport Si/T atomique global est compris entre 5 et 100 et de préférence entre 6 et 80 et de manière encore plus préférée entre 8 et 60, et dont le rapport Si/T de charpente est tel que le nombre d'atomes T extra-réseau est inférieur à 15%, et de préférence à 10 %, du nombre d'atomes T total (c'est à dire la somme du nombre d'atomes T extra-réseau et du nombre d'atomes T de la charpente cristalline) compris dans ladite zéolithe.

L'invention concerne aussi un catalyseur comportant au moins une zéolithe de type structural mazzite au moins en partie sous forme acide, de préférence pratiquement totalement, sous forme acide, comprenant du silicium et au moins un élément T choisi dans le groupe formé par le gallium et l'aluminium, de préférence l'aluminium, dont le rapport Si/T atomique global est compris entre 5 et 100, de préférence entre 6 et 80, et de manière encore plus préférée entre 8 et 60, et dont le rapport Si/T de charpente est tel que le nombre d'atomes T extra-réseau est inférieur à 15%, et de préférence à 10%, du nombre d'atomes T total compris dans ladite zéolithe, éventuellement au moins un élément choisi dans l'ensemble formé par les groupes IB et VIII de la classification périodique des éléments et au moins une matrice (ou liant).

La matrice est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium et les silice-alumines, de préférence parmi les éléments du groupe formé par les alumines et les argiles.

La zéolithe de type structural mazzite selon l'invention est généralement choisie dans le groupe formé par la zéolithe oméga, la mazzite, la zéolithe LZ-202, la zéolithe mazzite gallosilicate ou la zéolithe ZSM-4, de préférence la zéolithe oméga, dont le diamètre des pores principaux est d'environ 7,4 Å et qui posséde un réseau microporeux monodimensionnel ("Atlas of Zeolites Structure Types", W.M. Meier et D.H. Olson, 3ème Edition, 1992).

Lorsqu'elle est comprise dans le catalyseur selon l'invention, la zéolithe de type structural mazzite est au moins en partie, de préférence pratiquement totalement, sous forme acide, c'est-à-dire sous forme hydrogène (H⁺), la teneur en sodium de la zéolithe étant généralement inférieure à 0,6 % poids et de préférence inférieure à 0,1 % poids.

Le catalyseur selon l'invention contient généralement de 10 à 99%, et de préférence de 20 à 95%, de zéolithe de type structural mazzite au moins en partie sous forme acide. Dans le cas où le catalyseur de la présente invention contient au moins un élément choisi dans l'ensemble formé par les groupes IB et VIII de la classification périodique des éléments, la teneur pondérale du(des)dit(s) élément(s) est généralement comprise entre 0,01 et 10%, de préférence entre 0,05 et 7%, et de façon encore plus préférée entre 0,10 et 5%. Le complément à 100 % poids consiste généralement en la part de matrice dans le catalyseur.

L'invention concerne aussi la préparation de ladite zéolithe de type structural mazzite et dudit catalyseur.

Pour préparer la zéolithe de type structural mazzite selon l'invention, dans le cas préféré où T est Al, on opère une désalumination d'une zéolithe de type structural mazzite brute de synthèse par toute méthode connue de l'homme du métier, en particulier selon le mode opératoire décrit dans le brevet US-A-4.780.436 lorsque T est l'aluminium, c'est-à-dire que l'on procède à une étape de calcination sous flux d'air sec, qui a pour but d'éliminer le structurant organique occlus dans la microporosité de la zéolithe, à au moins un échange ionique par au moins une solution de NH₄NO₃, de manière à éliminer pratiquement tout cation alcalin, en particulier le sodium, présent en position cationique dans la zéolithe, puis à au moins un cycle de désalumination de charpente, comportant au moins une calcination en présence de vapeur d'eau, à une température généralement comprise entre 550 et 850 °C, suivie d'au moins une attaque acide. Cependant, on adapte les conditions de calcination en présence de vapeur d'eau (température, pression de vapeur d'eau et durée du traitement) ainsi que les conditions d'attaque acide post-calcination (durée de l'attaque, concentration de l'acide utilisé et le rapport entre le volume d'acide et la masse de zéolithe), de manière à ce que le nombre d'atomes Al extra-réseau résiduel soit inférieur à 15 % du nombre total d'atomes Al présents dans la zéolithe.

Dans le cas préféré où T est Al, le cycle de désalumination de la charpente, comportant au moins une étape de calcination sous vapeur d'eau et au moins une étape d'attaque en milieu acide de la zéolithe de type structural mazzite, peut être répété autant de fois qu'il est nécessaire pour obtenir la zéolithe de type structural mazzite désaluminée possédant les caractéristiques désirées. De même, suite au traitement de calcination sous vapeur d'eau, plusieurs attaques acides successives, avec des solutions en acide de concentration différentes, peuvent être opérées.

Dans le cas préféré où T est Al, le nombre d'atomes d'aluminium présents dans la charpente de la zéolithe de type structural mazzite peut être déterminé par RMN du solide du noyau ²⁹Si avec rotation à l'angle magique suivi d'une déconvolution des pics, ou bien par spectrométrie infrarouge qui, par enregistrement des fréquences de vibration des éléments constitutifs de la charpente de la zéolithe de type structural mazzite permet la détermination du rapport Si/Al de charpente ou de réseau, et par conséquent la détermination du nombre d'atomes d'aluminium présents dans le réseau. Lorsque T est l'aluminium, les bandes de vibration sur lesquelles sont effectuées les mesures sont la bande de vibration de valence asymétrique, interne aux tétraèdres T'O₄, T' étant soit un atome de silicium soit un atome d'aluminium (entre 1010 et 1100 cm⁻¹) et la bande de vibration externe aux tétraèdres T'O₄ étant comprise entre 610 et 660 cm⁻¹.

La préparation du catalyseur être effectuée selon toute méthode connue de l'homme du métier. En général, elle est obtenue par mélange de la matrice et de la zéolithe puis par mise en forme. L'élément éventuel de l'ensemble formé par les groupe lB et VIII de la classification périodique des éléments peut être intoduit soit avant la mise en forme, ou bien lors du mélange, ou bien sur la zéolithe elle même avant de la mélanger, soit, de préférence, après la mise en forme. La mise en forme est généralement suivie d'une calcination, généralement à une température comprise entre 250 et 600 °C. L'élément éventuel de l'ensemble formé par les groupe IB et VIII de la classification périodique des éléments peut être intoduit après ladite calcination. Dans tous les cas, ledit élément est généralement déposé au choix soit, de préférence, pratiquement totalement sur la zéolithe, soit pratiquement totalement sur la matrice, soit en partie sur la zéolithe et en partie sur la matrice, ce choix s'effectuant, de la manière qui est connue de l'homme du métier, par les paramètres utilisés lors dudit dépôt, comme par exemple la nature du précurseur choisi pour effectuer ledit dépôt.

L'élément des groupes IB ou VIII, de préférence choisi dans le groupe formé par Ag, Ni et Pt, et de façon encore plus préférée Ni, peut également éventuellement être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Dans le cas de l'échange ionique à partir de précurseurs à base d'argent, de nickel ou de platine, on utilise habituellement des sels d'argent tels que les chlorures ou les nitrates, un complexe tétramine du platine, ou des sels de nickel tels que les chlorures, les nitrates, les acétates ou les formiates. Cette technique d'échange cationique peut également être utilisée pour déposer directement le métal sur la poudre de zéolithe, avant son mélange éventuel avec une matrice.

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécéssaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques minutes à 100 °C en étuve et après calcination, par exemple pendant 2 heures à 400 °C, l'élément éventuel, par exemple le nickel, peut être déposé, par exemple par échange ionique, ledit dépôt étant suivi d'une calcination finale, par exemple pendant 2 heures à 400 °C.

La mise en forme du catalyseur selon l'invention est généralement telle que le catalyseur est de préférence sous forme de pastilles, d'aggrégats, d'extrudés ou de billes, en vue de son utilisation.

La préparation du catalyseur se termine généralement par une calcination, dite calcination finale, habituellement à une température comprise entre 250 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination.

Le dépôt éventuel d'élément (des éléments) des groupes IB et VIII est suivi en général d'une calcination sous air ou oxygène, généralement entre 300 et 600°C, de préférence entre 350 et 550°C, et pour une durée comprise entre 0,5 et 10 heure(s), de préférence entre 1 et 4 heure(s). On peut procéder ensuite à une réduction sous hydrogène, généralement à une température comprise entre 300 et 600°C, de préférence entre 350 et 550°C, et pour une durée comprise entre 1 et 10 heure(s), de préférence entre 2 et 5 heures, de façon à obtenir ledit élément principalement sous forme réduite nécessaire à l'activité catalytique.

L'invention concerne aussi l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et d'hydrocarbures alkylaromatiques généralement en C₉⁺ (c'est à dire à au moins 9 atomes de carbone par molécule), telle que la transalkylation et/ou la dismutation de toluène et/ou d'alkylaromatiques C₉⁺ pour produire des xylènes. La charge d'un tel procédé peut comprendre de 0 à 100 % d'alkylaromatiques en C₉⁺ et de 0 à 100 % de toluène. Ledit catalyseur se révèle très efficace pour ladite utilisation. Le catalyseur composite de la présente invention se révèle être particulièrement stable, même en présence de charges à traiter contenant une grande quantité d'aromatiques lourds AC9(+).

Les conditions opératoires sont généralement les suivantes : une température comprise entre 250 et 600°C et de préférence entre 330 et 500°C ; une pression comprise entre 10 et 60 bar et de préférence entre 20 et 45 bar; une vitesse spatiale d'alimentation, exprimée en kilogramme de charge introduite par kilogramme de catalyseur et par heure, comprise entre 0,1 et 10 et de préférence entre 0,5 et 4 ; un rapport molaire hydrogène sur hydrocarbures compris entre 2 et 20 et de préférence entre 3 et 12.

Les exemples suivants illustrent l'invention sans toutefois en limiter la portée.

### Exemple 1 : Préparation de la zéolithe 1 selon l'invention

La matière première utilisée est une zéolithe oméga, qui possède un rapport atomique Si/Al global égal à 3,2, une teneur pondérale en sodium par rapport au poids en zéolithe oméga sèche d'environ 5,3 %, un volume de maille élémentaire de 2,196 nm³, et un volume poreux, à l'azote, mesuré à -196°C et à P/Pₒ=0,19 de 0,125 cm³ liquide par gramme.

Cette zéolithe oméga subit tout d'abord une calcination dite sèche à 550°C sous flux d'air et d'azote durant 6 heures. Puis le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 625°C, durant 4 heures. La zéolithe subit alors une attaque acide, à l'aide d'acide nitrique 1,5N, à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga (Ω1) sous forme H a un rapport Si/Al atomique global égal à 11,3, un rapport Si/Al de charpente, déterminé par RMN du ²⁹Si de 12, une teneur pondérale en sodium par rapport au poids de zéolithe oméga sèche de 160 ppm, un volume de maille élémentaire de 2,145 nm³, et une capacité d'adsorption en azote mesurée à -196°C et à P/Pₒ=0,19 de 0,214 cm³ liquide/g.

### Exemple 2 : Préparation du catalyseur Ω2 conforme à l'invention

La zéolithe Ω1 obtenue à l'exemple 1 est ensuite mise en forme par extrusion avec un gel d'alumine de manière à obtenir, après séchage et calcination sous air sec, le catalyseur Ω2 qui contient en poids 80 % de zéolithe oméga et 20 % d'alumine.

### Exemple 3 : Préparation du catalyseur Ω3 conforme à l'invention

Dans cet exemple, le catalyseur Ω2 préparé dans l'exemple 2, est soumis à 3 échanges ioniques avec une solution d'acétate de nickel de manière à introduire 1,0% poids de nickel dans le catalyseur.

Pour cela, le catalyseur Ω2 est mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est, pour chaque échange, ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur Ω3 ainsi obtenu contient, en % poids, 79,30 % de zéolithe oméga forme hydrogène exempte d'espèces aluminiques extra-réseau, 19,80 % d'alumine et 0,85 % de nickel.

### Exemple 4 : Préparation de la zéolithe Ω4 non conforme à l'invention

La matière première utilisée est la même zéolithe oméga que celle utilisée dans l'exemple 1.

Puis cette zéolithe oméga subit tout d'abord une calcination dite sèche à 550°C sous flux d'air et d'azote durant 6 heures. Puis, le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 625°C, durant 4 heures. La zéolithe subit alors une attaque acide, à l'aide d'acide nitrique 1N, à environ 100°C, pendant 3,5 heures, de manière à extraire partiellement les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga (Ω4) sous forme H a un rapport Si/Al atomique global égal à 6,5, un rapport Si/Al de charpente, déterminé par RMN du ²⁹Si de 12,1, une teneur pondérale en sodium par rapport au poids de zéolithe oméga sèche de 155 ppm, un volume de maille élémentaire de 2,147 nm³, et une capacité d'adsorption en azote mesurée à -196°C et à P/Pₒ=0,19 de 0,195 cm³ liquide/g.

### Exemple 5 : Préparation de la zéolithe Ω5 non conforme à l'invention

La matière première utilisée est la même zéolithe oméga que celle utilisée dans l'exemple 1.

Néanmoins, dans cet exemple la zéolithe oméga ne subit pas de désalumination. Elle est soumise dans un premier temps une calcination dite sèche à 550°C sous flux d'air et d'azote durant 6 heures. Puis, le solide obtenu est soumis à trois échanges ioniques dans une solution de NH₄NO₃ 10N, à environ 100°C pendant 4 heures pour chaque échange. La forme ammonium de la zéolithe oméga ainsi obtenue est alors calcinée dans un premier temps sous flux d'air sec dilué dans l'azote (0,4l/h/g d'air sec pour 2l/h/g d'azote) durant 2 heures, puis uniquement sous flux d'air sec durant 4 heures à la température de 550°C.

A l'issue de ces traitements, la zéolithe oméga (Ω5) sous forme H ainsi obtenue posséde un rapport Si/Al atomique global égal à 3,2, un rapport Si/Al de charpente, déterminé par RMN du ²⁹Si de 3,3, une teneur pondérale en sodium par rapport au poids de zéolithe oméga de 150 ppm, un volume de maille élémentaire de 2,207 nm³, et une capacité d'adsorption en azote mesurée à -196°C et à P/Pₒ=0,19 de 0,22 cm³ liquide/g.

### Exemple 6 : Préparation des catalyseurs C1 et C2 (non conformes)

Les zéolithes non conformes à l'invention Ω4 et Ω5, sont mises en forme par extrusion avec un gel d'alumine de manière à faciliter leur manipulation et leur chargement dans un réacteur. Les catalyseurs extrudés correspondants sont notés C1 et C2, et contiennent tous deux 20 % poids d'alumine.

Un synopsis des compositions des catalyseurs Ω2 de l'exemple 2, et Ω3 de l'exemple 3, C1 et C2 de l'exemple 6, est donné dans le tableau suivant.

| Catalyseurs | Zéolithe **oméga** (% poids) | **Nickel** (% poids) | **Alumine** (% poids) |
|---|---|---|---|
| **Ω2** selon l'invention | 80,00 | 0,0 | 20,0 |
| **Ω3** selon l'invention | 79,32 | 0,85 | 19,83 |
| **C1** | 80,0 | 0,0 | 20,0 |
| **C2** | 80,0 | 0,0 | 20,0 |

### Exemple 7 : Evaluation des performances des catalyseurs

Les catalyseurs sont mis en oeuvre dans un réacteur à lit fixe, sous pression, dans lequel est introduit la charge qui est constituée de toluène pur.

La comparaison des rendements en (benzène + ethylbenzène + xylènes) obtenus en utilisant les catalyseurs Ω1 et Ω2, conformes à l'invention, et C1 et C2, non conformes à l'invention, est effectuée dans le tableau ci-après :

| Catalyseurs | Ω1 (conforme) | C1 (non conforme) | Ω2 (conforme) | C2 (non conforme) |
|---|---|---|---|---|
| Température de réaction (°C) | 450 | 450 | 450 | 450 |
| Pression totale de réaction (Bar) | 30 | 30 | 30 | 30 |
| Rendements % poids (Benzène + Ethylbenzène + xylènes) | 38,0 | 36,9 | 38,0 | 34,5 |

La comparaison des catalyseurs Ω1 et Ω2 et d'autre part la comparaison des catalyseurs C1 et C2, montre que les catalyseurs selon l'invention, Ω1 et Ω2, conduisent à des rendements en (Benzène + Ethylbenzène + xylènes) supérieurs à ceux obtenus avec les catalyseurs non conformes C1 et C2.

## Revendications

1. Zéolithe de type structural mazzite, comprenant du silicium et au moins un élément T choisi dans le groupe formé par le gallium et l'aluminium, dont le rapport Si/T atomique global est compris entre 5 et 100, et dont le rapport Si/T de charpente est tel que le nombre d'atomes T extra-réseau est inférieur à 15% du nombre d'atomes T total compris dans ladite zéolithe.

2. Zéolithe selon la revendication 1 telle que le nombre d'atomes T extra-réseau est inférieur à 10% du nombre d'atomes d'atomes T total compris dans l'ensemble de ladite zéolithe.

3. Zéolithe selon l'une des revendications 1 ou 2 telle que T est l'aluminium.

4. Zéolithe selon l'une des revendications 1 à 3 choisie dans le groupe formé par la zéolithe mazzite gallosilicate, la mazzite, la zéolithe LZ-202, la zéolithe oméga ou la zéolithe ZSM-4.

5. Zéolithe oméga selon l'une des revendications 1 à 4.

6. Préparation de la zéolithe selon l'une des revendications 1 à 5 à partir d'une zéolithe de type structural mazzite brute de synthèse par au moins un cycle d'extraction d'élément T de la charpente zéolithique, comportant au moins une calcination en présence de vapeur d'eau suivie d'au moins une attaque acide.

7. Préparation selon la revendication 6 telle que l'extraction est une désalumination de la charpente.

8. Catalyseur comportant au moins une zéolithe de type structural mazzite selon l'une des revendications 1 à 5 ou préparée selon la revendication 6 ou 7, au moins en partie sous forme acide, éventuellement au moins un élément choisi dans l'ensemble formé par les groupes IB et VIII de la classification périodique des éléments et au moins une matrice.

9. Catalyseur selon la revendication 8 tel que la zéolithe est pratiquement totalement sous forme acide.

10. Catalyseur selon l'une des revendications 8 ou 9 tel que ledit élément est choisi dans le groupe formé par Ag, Pt et Ni.

11. Catalyseur selon l'une des revendications 8 à 10 comprenant de 10 à 99% de zéolithe et, dans le cas où ledit catalyseur contient au moins un élément choisi dans l'ensemble formé par les groupes IB et VIII de la classification périodique des éléments, entre 0,01 et 10% dudit élément, le complément à 100 % poids consistant en la part de matrice dans le catalyseur.

12. Catalyseur selon l'une des revendications 8 à 11 sous forme de pastilles, d'aggrégats, d'extrudés ou de billes.

13. Préparation du catalyseur selon l'une des revendications 8 à 12 comportant le mélange de la matrice et de la zéolithe puis la mise en forme, ainsi que le dépôt éventuel dudit élément.

14. Préparation du catalyseur selon la revendication 13 comportant une calcination finale à une température comprise entre 250 et 600 °C.

15. Utilisation du catalyseur selon l'une des revendications 8 à 12 ou préparé selon l'une des revendications 13 ou 14 en dismutation d'hydrocarbures alkylaromatiques et/ou en transalkylation d'hydrocarbures alkylaromatiques.

16. Utilisation selon la revendication 15 en dismutation et/ou en transalkylation de toluène et/ou d'alkylaromatiques comprenant au moins 9 atomes de carbone par molécule.
